# EUROPEAN PATENT APPLICATION

(11) **EP 0 611 607 A1**
(43) Date of publication of application: **24.08.1994**
(21) Application number: 94100373.3
(22) Date of filing: 12.01.1994
(51) Int. Cl.: B05C 1/16, A61F 13/15

(54) **Process and apparatus for applying a coating to the surface of a web**

(30) Priority: 16.02.1993 US 17870
(71) Applicant: AVGOL LTD., IL-58101 Holon (IL)
(72) Inventor: Goldwasser, Moshe D., Tel Aviv 69499 (IL)
(74) Representative: Finck, Dieter, Dr.Ing.

(57) **Abstract**

A process and an apparatus are provided for unwinding an integrated web (W) of woven or nonwoven, hydrophobic or hydrophyllic coverstock material for baby diapers or other similar sanitary disposable absorbent products, such as sanitary napkins, adult products and the like, feeding that web (W) through a press or calender roll (23,24) to provide a uniform structure, and thereafter applying, a surfactant (or hydrophobicity-inducing) material (26) in selective areas of the web. Thereafter, the web may be passed through a drying system (33) so as to set or cure a coating material. The treated web may be wound in a roll (35) which is subsequently used on a diaper-making machine, or it may be fed directly to a diaper machine on which a partially assembled baby diaper is being made. The treated areas are placed accurately on an appropriate area of the diaper.

## Description

The invention relates to a process and apparatus for applying a coating of selected material to the surface of a well-integrated thin flexible porous web.

It is well known in the art for the manufacture of disposable baby diapers or other sanitary disposable absorbent products such as sanitary napkins, adult incontinence pads, and hospital bed pads to provide a structure wherein the coversheet or coverstock or top sheet, i.e. that portion of the product which is in contact with the patient's skin, is made of hydrophyllic material such as rayon, so as to be pervious to liquids such as urine. This permits ready pass-through of the liquid into the absorbent core or the pad which lies directly beneath the coverstock. In the past, some of the coverstocks have also been made of cotton, or blends of cotton and rayon or blends of rayon with a bonding fiber such as polyethylene.

Such coverstock, however, has been undesirable because it tends to retain the moisture and thus feel wet to touch and because it keeps the skin wet and is more likely to cause skin rash or diaper rash or the like.

Therefore, in more recent years, it has been found desirable to use a coverstock made of hydrophobic fibers or filaments, such as polypropylene or polyester, either carded, spun-bonded or melt-blown, or the like, but because such material inherently tends to interfere with the pass-through of urine into the absorbent pad, it has been found necessary to treat the web with a hydrophyllicity-inducing material such as a surfactant.

In order to do this, the diaper is provided with a continuous strip of hydrophobic material which has been over-all coated with a surfactant to make it pervious to urine. The coverstock web thus formed is laid continuously on the diaper as it is constructed on the diaper-making machine, but much of the web has been coated in areas where perviousness is not only unnecessary but also undesirable. For reasons of economy, the web is usually made of spun-bonded polypropylene or a carded thermo-bonded polypropylene or polyester fiber. In a more recent form of diaper construction, a separate web of hydrophyllic material is assembled between two webs made of hydrophobic fibers. This enables the coverstock material, when placed upon a diaper, with the pervious strip generally in the center of the diaper, to have the advantage of permitting the urine to pass through, but also to resist the transfer of urine along the sides of the coverstock and, therefore, contained within the outer edges of the diaper. However, this pre-formed web is costly to make and difficult to run on the diaper machine.

Moreover, as attractive as such a process might be, it is not completely satisfactory, because it uses an excess of surfactant, and thus is unnecessarily adding to the cost of the baby diaper, which cost should be kept as low as possible.

It is the object of the invention to overcome such deficiencies. This invention provides a process for applying a surfactant material only where desired in the coverstock.

Although such a process might be somewhat similar to the padding process which applies adhesive to an unbonded web of fibers (to create an integrated nonwoven web), it is believed that such a system has not heretofore been applied to the treatment of an integrated coverstock, either non-woven or woven, of the thin, soft, low-weight type which is a component and expensive element of a disposable baby diaper or adult incontinence pad.

Furthermore, it is believed that such a system has never been used directly in conjunction with the operation of the machine which produces the baby diaper or adult pad (as contrasted to an "off-line" system which prepares rolls of coverstock material for later use on the diaper-making machine).

Thus, the prior art fails to teach how a coverstock with uniquely-applied hydrophyllic zones can be produced for disposable absorbent sanitary products.

The object of the invention is achieved in accordance with the subjects of the attached claims.

In the present invention there is provided a process and apparatus for unwinding an integrated web of woven or nonwoven, hydrophobic or hydrophyllic coverstock material for baby diapers or other similar sanitary disposable absorbent products, such as sanitary napkins, adult products and the like, feeding that web through a press or calender roll to provide a uniform structure, and thereafter applying a surfactant (or hydrophobicity-inducing) material in selective areas of the web. Thereafter, the web may be passed through a drying system so as to set or cure a coating material. The treated web may be wound in a roll which is subsequently used on a diaper-making machine, or it may be fed directly to diaper machine on which a partially assembled baby diaper is being made. The treated areas are placed accurately on an appropriate area of the diaper.

According to the invention a process for the application of surfactant substances to a web of nonwoven coverstock made of hydrophobic material so as to make selected areas of the web hydrophyllic, a process for applying hydrophobicity-inducing materials to selected areas of a web of hydrophyllic material so as to render the selected areas impervious to the passage of liquids, a one-piece web which has selected liquid-pervious areas for the coverstock of disposable baby diaper, and an on-line means for selectively treating a web of coverstock are provided as it is fed from a supply roll directly to the machine for producing baby diapers wherein the selective zones have been carefully and precisely treated with a surfactant material to make an otherwise hydrophobic material hydrophyllic in the selective areas. Conversely, the treating material may be hydrophobicity-inducing so as to render a hydrophyllic web impervious to liquid in selected areas.

Embodiments of the invention are described referring to accompanying drawings, in which
Figure 1 is a schematic diagram of one form of apparatus and process for producing the web of the present invention.
Figure 2 is another schematic diagram similar to that of Figure 1 but showing how the web is treated selectively to render the "target area" of the diaper coverstock pervious to urine by producing discreet areas of hydrophyllicity (or hydrophobicity) and then placing the coverstock in proper registration on top of a partially assembled absorbent pad.

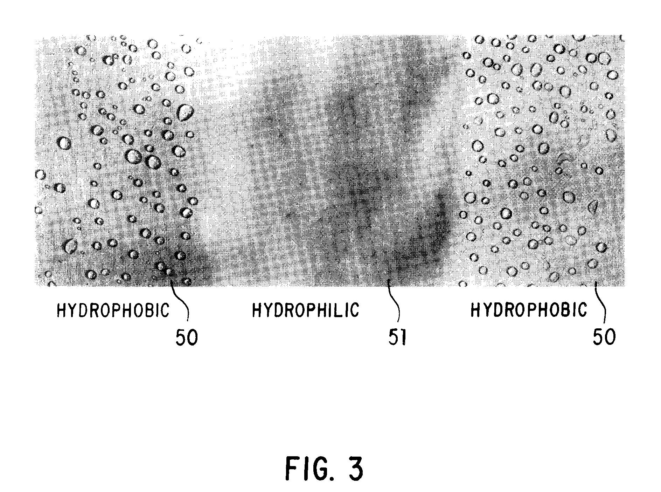

Figure 3 represents a reproduction of the surface of a hydrophobic web material which had areas treated to render it hydrophyllic, showing how the hydrophobic areas repel liquid while the hydrophyllic areas permit the liquid to pass through.
Figure 4 is a stylized perspective view of a "kiss" roll or "padding" roll showing how an enlarged portion thereof is developed so as to provide a surfactant material in discreet areas on a web passing over such roll.
Figure 5 illustrates a stylized view of a baby diaper showing a continuous urine-pervious strip on an otherwise hydrophobic web.
Figure 6 illustrates how the preferred hydrophyllic area can be precisely located so as to render the crotch portion and a portion of the waist hydrophyllic.
Figure 7 illustrates a view similar to Figures 5 and 6 but showing how the hydrophyllic area can be limited to the crotch portion of the diaper.
Figure 8 illustrates the hydrophyllic area is selectively located for a diaper intended for use by girls.
Figure 9 is similar to Figure 8 but shows how the area is located for a diaper which is intended for use by boys.

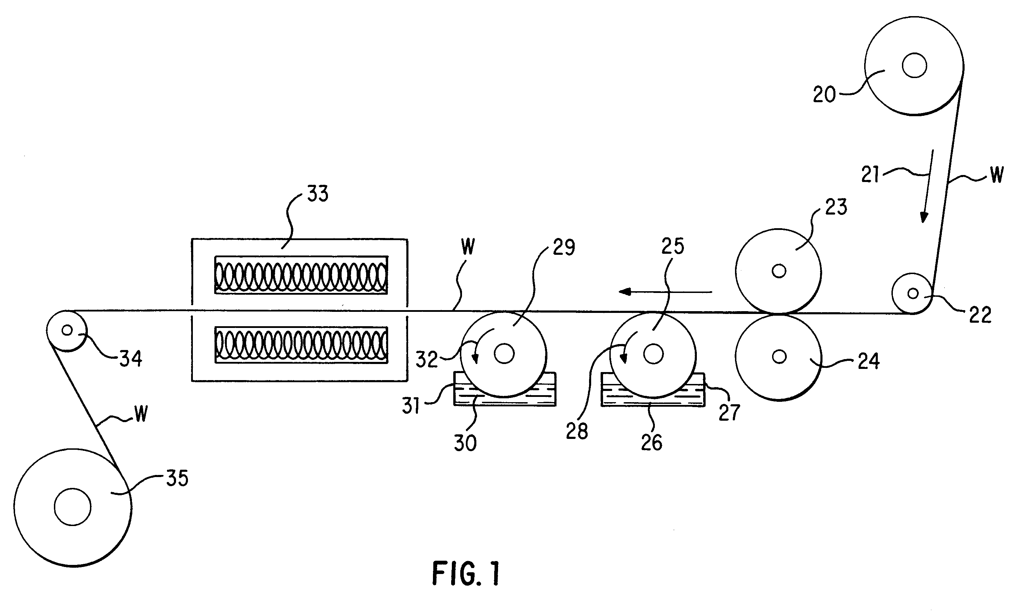

Referring now to Figure 1, there is shown a pre-formed, well-integrated roll 20 of nonwoven (or woven) coverstock material W which is unwound in the direction of the arrow 21 turning around a carrier roll 22 so that it can pass between rolls 23 and 24 which comprise a set of press or calender rolls.

Thereafter the web passes over a first "kiss" or "padding" roll 25 which applies selected material 26 from a pan or holder 27 when the roll 25 rotates within the material 26 in the direction of the arrow 28 . The material may be either a surfactant (if the web is hydrophobic in character) or a material which induces hydrophobicity (if the web is hydrophyllic).

If desirable, a second roll 29 can be used, also rotating in a bath 30 of material held within a pan 31 and rotating in the direction of the arrow 32 to apply a second coating. This second coating may be of the same material as applied in the first "kiss" roll or it may be opposite in character and applied in a different area of the web.

Thereafter, the web W passes through a drying chamber 33 and around a turning roll 34 to be rolled up on a winder 35 . The winder may be 120 inches wide and may be a combination winder/slitter so that individual rolls of finished coverstock of appropriate width may be produced. Desirable widths presently used in the industry are 15 inches, and on such 15 inch wide webs the central pervious strip may be 5 inches wide with a 5 inch wide strip of impervious material on each side of the central strip.

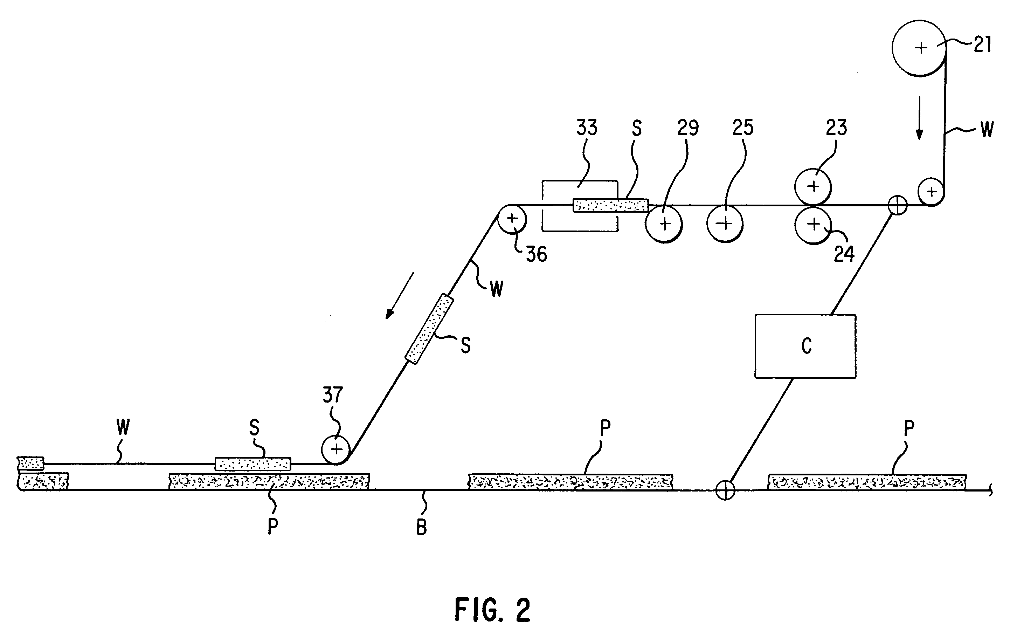

Another version of the invention is shown in Figure 2 wherein the web W is unwound from a roll 20 and passed between the calender rolls 23 and 24 and over the "kiss" rolls 25 and 29 and through a drying chamber 33 , and then turned around a carrier roll 36 downwardly to another turning roll 37 whereupon the pervious portion S of the web W is placed in proper, discreet alignment and registration on the absorbent pads P carried by a backing material B .

A control C is connected electronically with proper drive mechanism to the web W and to the backing B (or conversely whichever portion of the transport mechanism carries the web W or the backing B and the pad B ). This ensures that the pervious area S will always be in proper registration on the top of the pad P as the web W and the pad come together in the assembly portion of the baby diaper machine.

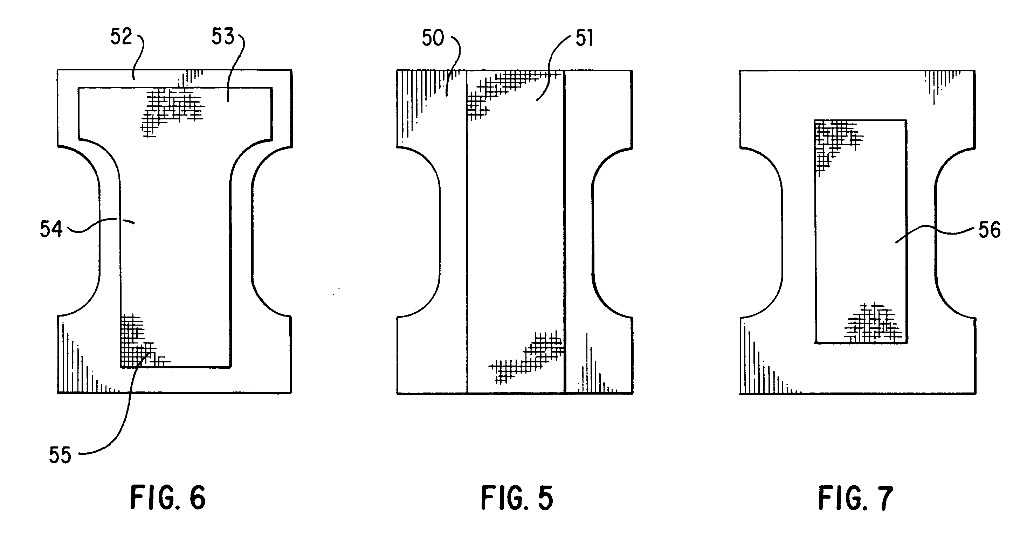

The application of the treating material may be continuous so as to provide an uninterrupted treated area (as shown in Figure 5). This "zebra-like" pattern may be preferred for a variety of reasons, not the least significant of which is ease and economy of operation, even though an excess of material may be applied in areas where its presence is not critical to the operation of the finished product.

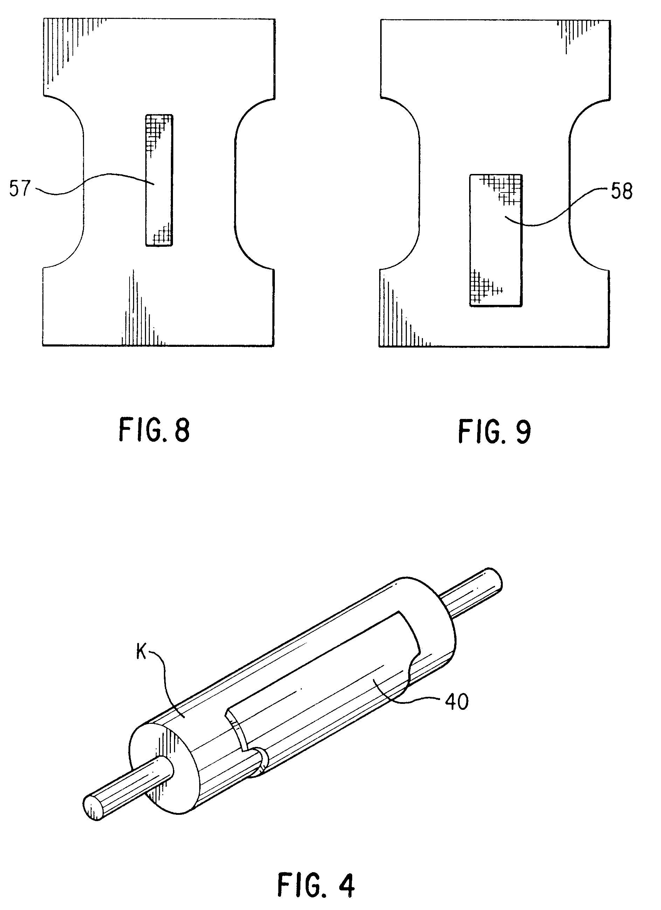

As is shown in Figure 4, a "kiss" or "padding" roll K has a raised portion 40 which picks up the material S from the carrier pan 27 or 31 , and the shape or pattern of the raised portion 40 may be chosen to provide the desired area of deposition. The illustration in Figure 4 is comparable to Figure 6 to show how the selective area 52 can be chosen so that the pervious area is created in the waist portion 53 , the crotch portion 54 , and toward the other end 55 of the diaper.

Figure 7 illustrates how the coating material can be limited to a rectangular area which stops short of the ends of the diaper.

Figure 8 illustrates how a smaller pervious area 57 can be selected for diapers to be used by girl babies, and Figure 9 illustrates how a selected area 58 can be chosen for diapers to be worn by boy babies.

Although a primary desire is to create a hydrophobic web material treated with a surfactant to render preferred areas hydrophyllic, it is to be understood that the reverse is just as possible, namely, that the web can be hydrophyllic in nature and areas outside the preferred area (such as the areas outside the portions 51 ; 52 ; 56, 57, 58 of Figures 5 to 9 inclusive) can be rendered hydrophobic, and thus the selected areas remain hydrophyllic because the web material itself was hydrophyllic to begin with.

The axles of the rolls 25 and 29 may be elliptical, or the axles may be supported on vertically oscillating trunnions so that the pattern 40 on the rolls may be raised into contact with selected areas of the web W and then lowered so that the coating material S is accurately applied only on the chosen areas of the web W .

## Claims

1. A process for applying a coating of selected material to the surface of a well-integrated thin flexible porous web, which process includes
- supplying a web of material,
- passing said web over an applying roll,
- providing said roll with a patterned area,
- rotating said roll in a supply of selected material so that said patterned area carries the selected material to the web when the roll is rotated,
- limiting the selected material on the web to an area defined by the pattern area.

2. The process of claim 1 wherein said web is a nonwoven made of hydrophobic material and said selected material renders the patterned areas pervious to the passage of liquid.

3. The process of claim 1 wherein said web is a nonwoven made of hydrophyllic material and said selected material renders the patterned areas impervious to the passage of liquid.

4. The process of claim 1 wherein said web is woven of hydrophyllic material and said selected material renders the patterned areas impervious to the passage of liquid.

5. The process of claim 2 wherein said web is spunbonded, melt blown or thermo-bonded.

6. The process of claim 3 or claim 4 wherein said web is made of cotton or of rayon.

7. The process according to one of the claims 1 to 6 wherein the process of coating the web takes place in synchronism with the operation of a diaper-making machine, and the web is applied directly to the diaper being made on the machine.

8. The process according to one of the claims 1 to 7 wherein the area of selected material on the web is discontinous.

9. The process of claim 7 wherein the area of the web containing the selected material overlies or does not overlie the crotch area of the diaper made on the machine.

10. The process according to one of the preceding claims wherein areas of the web containing the selected material are continuous and are between three and seven inches wide and are separated by continous areas of between eight and twelve inches wide which do not contain the selected material.

11. The process of claim 10 wherein the continous areas containing the selected material are each five inches wide and the continuous areas without the selected material are each ten inches wide.

12. The process according to one of the preceding claims wherein the web is finally wound up into a finished roll.

13. The process of claim 12 wherein the finished roll is slit into a plurality of narrow rolls.

14. The process of claims 11 to 13 wherein the web is wound up into a finished roll and the finished roll is slit into a plurality of narrow rolls in the middle of the continuous areas which do not have the selected material.

15. Apparatus for carrying out the method according to one of the claims 1 to 14.

16. Coated web produced according to one of the claims 1 to 14 or with an apparatus according to claim 15.
